# EUROPEAN PATENT APPLICATION

(11) **EP 3 789 076 A1**
(43) Date of publication of application: **10.03.2021**
(21) Application number: 19196227.3
(22) Date of filing: 09.09.2019
(51) Int. Cl.: A61M 39/10

(54) **CONNECTOR ELEMENT HAVING A GRIP ARRANGEMENT**

(71) Applicant: Fresenius Kabi Deutschland GmbH, 61352 Bad Homburg (DE)
(72) Inventor: BAUMGART, Steffen, 36088 Hünfeld (DE); PAWLOWSKI, Marc, 36404 Vacha (DE); IRAN, Benjamin, 36179 Bebra (DE)
(74) Representative: Fresenius Kabi Deutschland GmbH

(57) **Abstract**

A connector element (1, 2) for establishing a connection to another connector element (2, 1) comprises a body (10, 20) forming a connection portion (12, 22) for establishing a fluid connection with the other connector element (2, 1), and a grip arrangement (13, 23) which is axially displaced, along a longitudinal axis (L), with respect to the connection portion (12, 22). The connection portion (12, 22) is connectable to the other connector element (2, 1) by a screwing action (A1) in a screwing direction (S) about the longitudinal axis (L) and releasable from the other connector element (2, 1) by an unscrewing action (A2) in a direction opposite the screwing direction (S). Herein, the grip arrangement (13, 23) forms a first actuation face (133A, 133B) and a second actuation face (136A, 136B), wherein the first actuation face (133A, 133B) comprises a rounded curvature in a cross-sectional plane perpendicular to the longitudinal axis (L) and faces opposite the screwing direction (S) to enable a user action onto the grip arrangement (13, 23) for performing said screwing action (A1), and wherein the second actuation face (136A, 136B) faces in the screwing direction (S) to enable a user action onto the grip arrangement (13, 23) for performing said unscrewing action (A2).

## Description

The invention relates to a connector element for establishing a connection to another connector element according to the preamble of claim 1.

A connector element of this kind comprises a body forming a connection portion for establishing a fluid connection with the other connector element, and a grip arrangement which is axially displaced, along a longitudinal axis, with respect to the connection portion. The connection portion is connectable to the other connector element by a screwing action in a screwing direction about the longitudinal axis and releasable from the other connector element by an unscrewing action in a direction opposite the screwing direction.

A connector element of this kind may for example form part of a connector assembly serving to connect medical lines to each other. A medical line of this kind may for example be a line used for the enteral feeding of a patient. Enteral feeding generally refers to the delivery of a nutritionally complete feed, containing protein, carbohydrate, fat, water, minerals and vitamins, directly into the stomach, duodenum or jejunum of a patient. A feeding tube, for this purpose, may for example be passed through the nares (nostril), down the esophagus and into the stomach (so-called nasogastric feeding tube). A nasojejunal feeding tube, in comparison, may be passed further through the stomach into the jejunum, the middle section of the small intestine. A gastric feeding tube is a tube inserted through a small incision in the abdomen into the stomach and is used, preferably, for long-term enteral nutrition.

A connector element in this respect may for example form a male connector, for example a male connector according to ISO 80369-3. Alternatively, a connector element may form a female connector, for example a female connector according to ISO 80369-3.

Using such connector elements serving to establish a fluid connection for example in between medical lines, a screwing connection is established by screwing the connector elements onto each other. Herein, for the screwing action generally a sliding-friction must be overcome due to the interaction of screw threads of the connector elements with each other, requiring a comparatively small force.

For releasing the connector elements from each other, however, in addition to a sliding-friction potentially a stiction and adhesion may act in between the connector elements, such that a force exerted by a user onto the grip arrangement may have to be substantially larger for unscrewing the connector elements from each other.

There is a desire of a connector element that intuitively limits a force that a user exerts onto the grip arrangement for connecting connector elements to each other, while improving the handling for unscrewing connector elements from each other.

It is an object of the invention to provide a connector element that comprises a grip arrangement formed such that the handling of the connector element for connecting connector elements to each other as well as for releasing connector elements from each other is improved.

This object is achieved by means of a connector element comprising the features of claim 1.

Accordingly, the grip arrangement forms a first actuation face and a second actuation face. The first actuation face comprises a rounded curvature in a cross-sectional plane perpendicular to the longitudinal axis and faces opposite the screwing direction to enable a user action onto the grip arrangement for performing said screwing action. The second actuation face faces in the screwing direction to enable a user action onto the grip arrangement for performing said unscrewing action.

The second actuation face beneficially comprises a flat shape.

The grip arrangement hence forms different actuation faces allowing a user to act onto the grip arrangement for establishing a screwing connection to another connector element and for releasing the connector elements from each other. The actuation faces herein are shaped differently in order to improve an intuitive handling of the grip arrangement by a user.

Namely, a first actuation face comprises, in a cross-sectional plane perpendicular to the longitudinal axis of the connector element, a rounded shape. An action of a user on the first actuation face hence is somewhat weakened, in that a user with a finger may slide over the first actuation face and hence will act onto the first actuation face with a comparatively small actuation force. A connection of the connector element to another connector element, requiring to overcome substantially only a sliding friction, thus intuitively takes place at a comparatively small.

The first actuation face faces opposite the screwing direction, such that a user may act onto the first actuation face in the screwing direction in order to rotate the connector element by action onto the grip arrangement in the screwing direction.

A second actuation face in turn has e.g. a flat shape and faces in the screwing direction. A user may hence act onto the second actuation face in a direction opposite to the screwing direction in order to rotate the connector element for unscrewing the connector element from another connector element to which the connector element has been connected. For the releasing a sliding friction as well as a stiction and potentially an adhesion must be overcome, due to a usage of the connector element for establishing a flow connection to provide a passageway for a medical fluid. A force to be exerted on the grip arrangement for releasing the connector element from another connector element hence may be substantially larger than a force required for connecting the connector element to another connector element for establishing a connection. Due to the beneficially flat shape of the second actuation face a user with a finger may strongly press onto the grip arrangement such that a force can be easily exerted onto the grip arrangement for receiving the connector element.

In one embodiment, the second actuation face extends along a plane defined by a longitudinal direction pointing along the longitudinal axis of the connector element and a radial direction extending transverse to the longitudinal axis. The second actuation face hence is oriented with respect to the longitudinal axis such that a torque about the longitudinal axis may easily be introduced into the grip arrangement by acting onto the second actuation face. Due to the radial extension of the second actuation face a force may be efficiently transferred to the grip arrangement, such that the handling of the connector element for releasing the connection element from another connector element becomes easy and intuitive for a user.

Because the first actuation face and the second actuation face differ in their shapes, a user will experience a different handling in the screwing connection for establishing a connection versus opposite the screwing connection for releasing a connection. In this way, a torque exerted by a user in the screwing direction is intuitively limited, whereas a torque exerted by a user opposite the screwing direction is efficiently transferred to the grip arrangement and hence allows for an easy releasing of the connector element from another connector element.

In one embodiment, the first actuation face and the second actuation face adjoin each other at a first connection line. The first connection line herein may be oriented substantially along the longitudinal axis, wherein the first actuation face extends at a perpendicular angle from the second actuation face at the first connection line. This is to be understood in that the second actuation face forms a perpendicular angle to a tangent of the curved, first actuation face at the first connection line in the cross-sectional plane perpendicular to the longitudinal axis. The second actuation face hence extends from the first actuation face at a substantially right angle.

In one embodiment, the first actuation face is adjoined by an outer face extending about the longitudinal axis. The outer face may for example, in said cross-sectional plane, comprise a constant radius about the longitudinal axis and hence have the shape of an arc in said cross-sectional plane. The first actuation face herein may converge into the outer face such that the first actuation face smoothly transitions into the outer face.

In one embodiment, the outer face is adjoined, at an end opposite the first actuation face, by a further second actuation face at a second connection line. The further second actuation face may for example extend radially inwards from the outer face at the second connection line, and may form a perpendicular angle with the outer face at the second connection line. This again is to be understood in that the further second actuation face forms a perpendicular angle with a tangent of the outer face at the second connection line in the cross-sectional plane perpendicular to the longitudinal axis.

The second actuation faces may be diametrically opposite to one another such that a symmetric action onto the grip arrangement for releasing the connection element from another connector element may be exerted by a user.

Likewise, the first actuation face may be arranged diametrically opposite to another first actuation face, such that the first actuation faces are symmetrically arranged with respect to one another and a user may act onto the grip arrangement in a symmetric fashion in order to establish a connection with another connector element.

Generally, the grip arrangement, in said cross-sectional plane perpendicular to the longitudinal axis, may have a point-symmetric shape with respect to the longitudinal axis, such that a user, for example by using two fingers, may symmetrically and intuitively act onto the grip arrangement both for establishing and for releasing a connection.

It however - alternatively - is also conceivable that the grip arrangement forms only a single first actuation face and a single second actuation face and does not comprise a point-symmetry.

A connector assembly for connecting medical lines to each other, in one embodiment, comprises a connector element of the type described above and in addition another connector element which is connectable to the connector element. Herein, each connector element may be connected to a medical line, such that by connecting the connector elements to each other a fluid connection in between the medical lines may be established. Herein, both connector elements may have a grip arrangement of the form as described above, such that a user with two hands may grab the connector elements and may act onto the connector elements for establishing a connection in between the connector elements as well as for releasing a connection of the connector elements.

The idea underlying the invention shall subsequently be explained in more detail with reference to the embodiments shown in the figures. Herein:
- Fig. 1: shows a schematic drawing of a feeding line placed on a patient;
- Fig. 2: shows an embodiment of a connector assembly having connector elements;
- Fig. 3: shows a view of a connector element in the shape of a female connector;
- Fig. 4: shows a view of a connector element in the shape of a male connector;
- Fig. 5: shows another view of a connection element in the shape of a female connector;
- Fig. 6: shows a view of a grip arrangement of a connector element, according to the prior art;
- Fig. 7: shows a view of an embodiment of a grip arrangement of a connector element; and
- Fig. 8: shows a schematic side view of a grip arrangement of a connector element in the shape of a female connector.

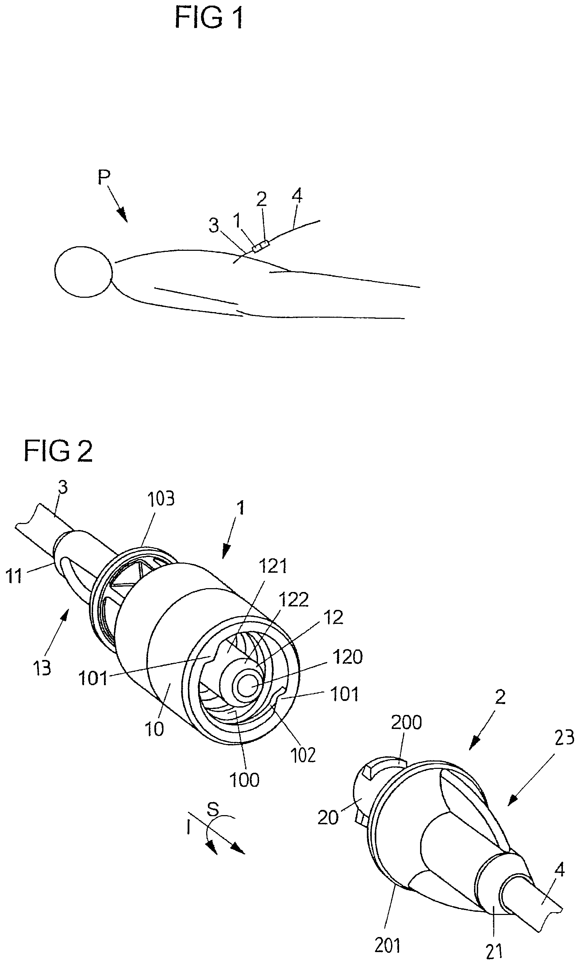

Fig. 1 shows a schematic drawing of a feeding line assembly comprising feeding lines 3, 4 placed on a patient P.

The feeding lines 3, 4 for example serve for providing for an enteral feeding of a patient P. For this, a first line 3 may, for example through an incision in the abdomen of the patient P, be inserted into the stomach of the patient P and may be used as a port for a long-term enteral feeding. A second feeding line 4 is connected to the first feeding line 3 via a connector assembly comprising connector elements 1, 2, the second feeding line 4 for example being connected to a container comprising an enteral feeding solution, which via the lines 3, 4 is delivered towards the patient P for example using a suitable pumping device.

An embodiment of a connector assembly comprising connector elements 1, 2 is shown in Fig. 2. A first connector element 1 herein may for example be connected to a feeding line 3 placed on a patient P, whereas a second connector element 2 is arranged on another, second feeding line 4 connected to a container comprising a feeding solution.

The first connector element 1, in this embodiment, comprises a cylindrical body 10 circumferentially coaxially extending about a connection portion in the shape of a cylindrical or conical insertion shaft 12 and forming, together with the insertion shaft 12, a radial inner space 102. On the inner wall of the body 10 a screw thread 100 is arranged allowing for a screw type connection with the second connector element 2.

The insertion shaft 12 can be inserted in an insertion direction I into a connection portion in the shape of an insertion opening 22 in a body 20 of the second connector element 2. On the body 20 of the second connector element 2 a screw thread 200 is arranged, such that the second connector element 2 can be screwed into the inner space 102 of the first connector element 1 by engaging the screw threads 100, 200 with each other and by screwing the first connector element 1 along a screwing direction S on the second connector element 2.

The insertion shaft 12 has a substantially cylindrical or conical shape having a circular cross section. The insertion opening 22 of the second connector element 2 has a corresponding, complementary shape and hence is suited to receive the insertion shaft 12.

When screwing the connector elements 1, 2 on to each other, the insertion shaft 12 is inserted into the insertion opening 22. The insertion shaft 12 comprises a fluid channel 120, which extends through the insertion shaft 12 and through a shaft 11 aligned with the insertion shaft 12, the shaft 11 being connected to the associated feeding line 3. Likewise, the insertion opening 22 is aligned with a shaft 21 of the second connector element 2, the shaft 21 being connected to the feeding line 4. Hence, by connecting the connector elements 1, 2 to each other, a fluid connection between the feeding lines 3, 4 can be established, such that a medical fluid such as an enteral feeding solution may pass from the feeding line 4 through the connector elements 1, 2 into the feeding line 3.

The connector elements 1, 2 each may for example be fabricated from a comparatively hard plastics material, for example by injection molding.

The connector elements 1, 2 of the example of Fig. 2 are connectable by screwing the connector elements 1, 2 on to each other along a screwing direction S, and may be released from one another by an unscrewing action opposite the screwing direction S. Herein, each connector element 1, 2 comprises a grip arrangement 13, 23 arranged at an end of the connector element 1, 2 opposite the connection portion in the shape of the insertion shaft 12 respectively the insertion opening 22 and adjoining a rim 103, 201 of the respective connector element 1, 2. Using the grip arrangement 13, 23 a user may manually grip the connector elements 1, 2 and may screw the connector elements 1, 2 onto one another or release the connector elements 1, 2 from another.

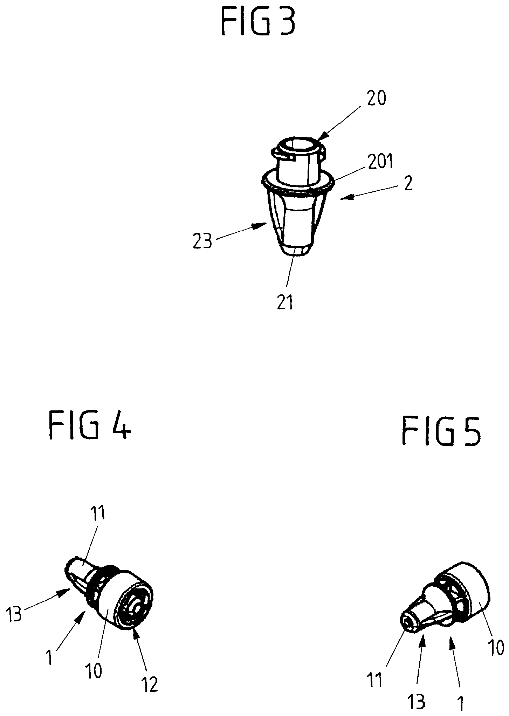

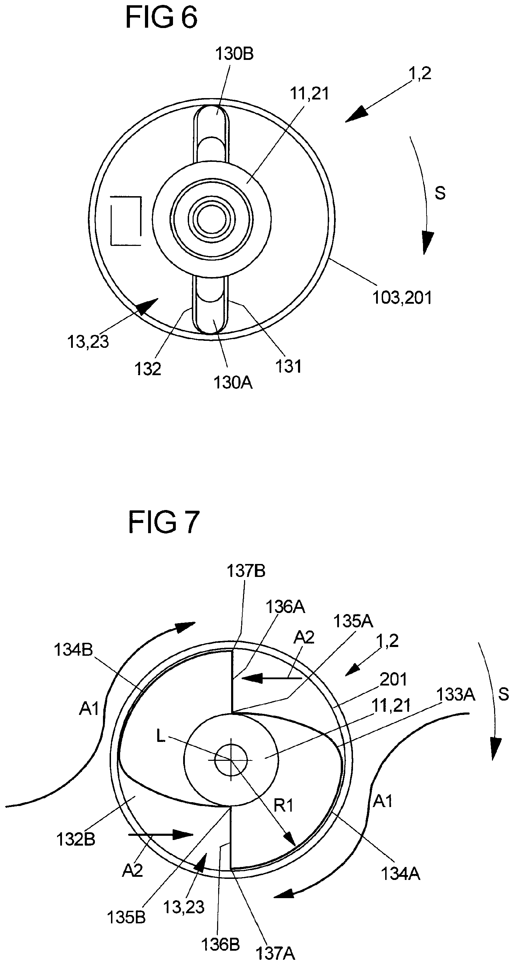

Fig. 3 to 5 show views of connector elements 1, 2 in the shape of a female connector (Fig. 3) and a male connector (Figs. 4 and 5) according to ISO 80369-3. As illustrated in Fig. 6, a grip arrangement 13, 23 of such connector elements 1, 2 conventionally comprises two wing sections 130A, 130B having a symmetric shape with opposing, flat faces 131, 132 for allowing a user to act onto the grip arrangement 13, 23 both in the screwing direction S and opposite the screwing direction S.

Generally, for establishing a connection in between connector elements 1, 2 a sliding-friction must be overcome, such that a user has to exert a comparatively small torque onto the connector elements 1, 2 for establishing the connection. In contrast, for releasing the connection of the connector elements 1, 2, a substantially larger torque may be required, as in addition to a sliding-friction a stiction force and an adhesion potentially must be overcome.

To address this, it herein is proposed to shape faces of a grip arrangement 13, 23 allowing a user to act onto a connector element 1, 2 for establishing a screw connection differently from faces which allow a user to act onto a connector element 1, 2 for releasing a connection.

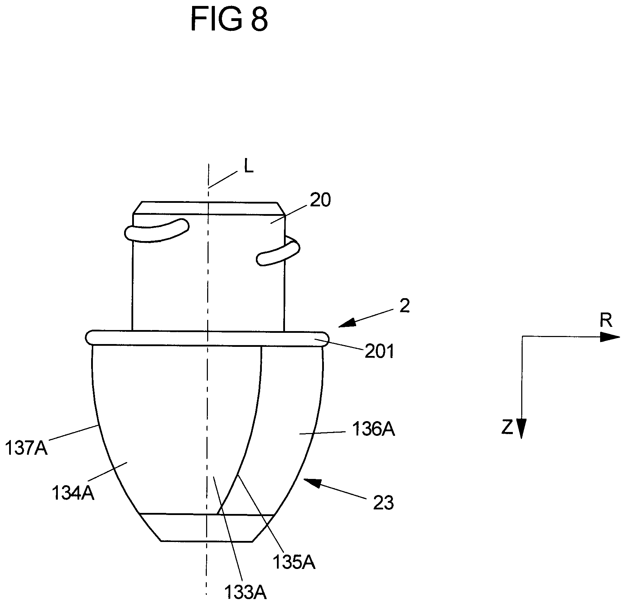

Referring now to Figs. 7 and 8, a grip arrangement 13, 23 of a connector element 1, 2 - in one embodiment - comprises first actuation faces 133A, 133B which are formed diametrically opposite to one another on the grip arrangement 13, 23 and have a rounded shape in a cross-sectional plane perpendicular to a longitudinal axis L of the connector element 1, 2, as shown in Fig. 7. The first actuation faces 133A, 133B each are defined by a radius which, starting from a first connection line 135A, 135B, continuously increases until the first actuation face 133A, 133B converges into an associated outer face 134A, 134B having a constant radius R1 with respect to the longitudinal axis L.

At a respective first connection line 135A, 135B, each first actuation face 133A, 133B adjoins a second actuation face 136A, 136B, the second actuation face 136A, 136B in the illustrated embodiment extending radially with respect to the longitudinal axis L from the associated first actuation face 133A, 133B.

Each second actuation face 136A, 136B, at a respective second connection line 137B, 137A, adjoins an associated outer face 134B, 134A.

The second actuation face 136A, 136B has a flat shape and extends along a plane defined by a longitudinal direction Z aligned with the longitudinal axis L and a radial direction R (see Fig. 8).

Each second actuation face 136A, 136B herein forms a right angle with a tangent to the associated first actuation face 133A, 133B at the first connection line 135A, 135B and with a tangent to the associated outer face 134B, 134A at the second connection line 137B, 137A.

The grip arrangement 13, 23, in the cross-sectional plane perpendicular to the longitudinal axis L, comprises a point-symmetric shape with respect to the longitudinal axis L, as visible from Fig. 7.

As visible from Fig. 8, the radial width and diameter of the faces 133A, 133B, 134A, 134B, 136A, 136B, may vary along the longitudinal axis L, such that the grip arrangement 13, 23 may have a reducing diameter towards an end of the connector element 1, 2 opposite the connection portion 12, 22.

The first actuation faces 133A, 133B face opposite the screwing direction S, as visible from Fig. 7. Hence, a user may act onto the first actuation faces 133A, 133B in order to exert a torque on the grip arrangement 13, 23 in the screwing direction S for establishing a screw connection with another connector element 2, 1 (screwing action A1 in Fig. 7). Due to the rounded shape of the first actuation faces 133A, 133B and the converging into the outer faces 134A, 134B, a user may slide over the actuation faces 133A, 133B, such that a user force in the screwing direction S is naturally limited and a user does not apply an excessive force for establishing the screw connection.

The second actuation faces 136A, 136B, in turn, face in the screwing direction S, as visible from Fig. 7. Hence, a user may act onto the second actuation faces 136A, 136B in a direction opposite the screwing direction S (unscrewing action A2 in Fig. 7). By acting onto the second actuation faces 136A, 136B a user may exert a torque onto the respective connector element 1, 2 in order to unscrew the connector element 1, 2 from another connector element 2, 1, wherein due to the flat shape of the second actuation faces 136A, 136B and due to the radial extension a force may efficiently be introduced into the grip arrangement 13, 23 and hence into the connector element 1, 2.

Hence, a larger force may be applied by a user for unscrewing the connector element 1, 2 from another connector element 2, 1, such that a user may easily handle the connector element 1, 2 for overcoming a sliding friction as well as a stiction and adhesion.

The invention is not limited to the embodiments described above, but may be implemented in an entirely different fashion.

For example, a connector element of the kind described herein may not only be used for connecting medical lines for the purpose of an enteral feeding, but may be used for connecting any medical connector elements for transferring medical solutions, such as medication, nutritional solutions, saline solutions or any other solutions.

### List of Reference Numerals

- 1: Connector (male part)
- 10: Body
- 100: Screw thread
- 101: Protrusion
- 102: Space
- 103: Rim
- 11: Shaft
- 12: Connection portion (insertion shaft)
- 120: Fluid channel
- 121: Surface (outer wall)
- 122: Tip
- 13: Grip arrangement
- 130A, 130B: Wing section
- 131A-133A, 131B-133B: Actuation face
- 134A, 134B: Outer face
- 135A, 135B, 137A, 137B: Connection line
- 136A, 136B: Actuation face
- 2: Connector (female part)
- 20: Body
- 200: Screw thread
- 201: Rim
- 21: Shaft
- 22: Connection portion (insertion opening)
- 23: Grip arrangement
- 3: Line
- 4: Line
- A1: Screwing action
- A2: Unscrewing action
- I: Insertion direction
- L: Longitudinal axis
- P: Patient
- R: Radial direction
- R1: Radius
- S: Screwing direction
- Z: Longitudinal direction

## Claims

1. Connector element (1, 2) for establishing a connection to another connector element (2, 1), comprising: a body (10, 20) forming a connection portion (12, 22) for establishing a fluid connection with the other connector element (2, 1), and a grip arrangement (13, 23) which is axially displaced, along a longitudinal axis (L), with respect to the connection portion (12, 22), wherein the connection portion (12, 22) is connectable to the other connector element (2, 1) by a screwing action (A1) in a screwing direction (S) about the longitudinal axis (L) and releasable from the other connector element (2, 1) by an unscrewing action (A2) in a direction opposite the screwing direction (S),
**characterized in**
**that** the grip arrangement (13, 23) forms a first actuation face (133A, 133B) and a second actuation face (136A, 136B), wherein the first actuation face (133A, 133B) comprises a rounded curvature in a cross-sectional plane perpendicular to the longitudinal axis (L) and faces opposite the screwing direction (S) to enable a user action onto the grip arrangement (13, 23) for performing said screwing action (A1), and wherein the second actuation face (136A, 136B) faces in the screwing direction (S) to enable a user action onto the grip arrangement (13, 23) for performing said unscrewing action (A2).

2. Connector element (1, 2) according to claim 1, **characterized in that** the second actuation face (136A, 136B) comprises a flat shape.

3. Connector element (1, 2) according to claim 1 or 2, **characterized in that** the second actuation face (136A, 136B) extends along a plane defined by a longitudinal direction (Z) pointing along the longitudinal axis (L) and a radial direction (R).

4. Connector element (1, 2) according to one of claims 1 to 3, **characterized in that** the first actuation face (133A, 133B) and the second actuation face (136A, 136B) adjoin each other at a first connection line (135A, 135B).

5. Connector element (1, 2) according to claim 4, **characterized in that** the first actuation face (133A, 133B) extends at a perpendicular angle from the second actuation face (136A, 136B) at the first connection line (135A, 135B).

6. Connector element (1, 2) according to one of the preceding claims, **characterized in that** the first actuation face (133A, 133B) is adjoined by an outer face (134A, 134B) extending about the longitudinal axis (L).

7. Connector element (1, 2) according to claim 6, **characterized in that** the outer face (134A, 134B), in said cross-sectional plane, comprises a constant radius (R1) about the longitudinal axis (L).

8. Connector element (1, 2) according to claim 6 or 7, **characterized in that** the outer face (134A, 134B) is adjoined by a further second actuation face (136B, 136A) at a second connection line (137A, 137B).

9. Connector element (1, 2) according to claim 8, **characterized in that** the further second actuation face (136B, 136A) extends radially inwards from the outer face (134A, 134B) at the second connection line (137A, 137B).

10. Connector element (1, 2) according to claim 8 or 9, **characterized in that** the further second actuation face (136B, 136A) forms a perpendicular angle with the outer face (134A, 134B) at the second connection line (137A, 137B).

11. Connector element (1, 2) according to one of the preceding claims, **characterized in that** the grip arrangement (13, 23), in said cross-sectional plane, is point-symmetric with respect to the longitudinal axis (L).

12. Connector assembly for connecting medical lines (3, 4) to each other, comprising a connector element (1, 2) according to one of the preceding claims and another connector element (2, 1) connectable to the connector element (1, 2).
